# NEW EUROPEAN PATENT SPECIFICATION

(11) **EP 0 003 172 B2**
(45) Date of publication and mention of the opposition decision: **19.03.1997**
(45) Mention of the grant of the patent: 07.10.1987
(21) Application number: 79300039.9
(22) Date of filing: 10.01.1979
(51) Int. Cl.: A61K 7/32, C11D 3/50

(54) **The use of a deodorant detergent composition for suppressing human body malodour**
Die Verwendung einer desodorierenden Reinigungsmittelzusammensetzung zur Unterdrückung von unangenehmen Körpergeruch
L'utilisation d'une composition détergente déodorante pour supprimer les odeurs corporelles désagréables

(30) Priority: 12.01.1978 GB 128678
(43) Date of publication of application: 25.07.1979
(73) Proprietor: UNILEVER PLC, London EC4P 4BQ (GB); UNILEVER N.V., 3013 AL Rotterdam (NL)
(72) Inventor: Hooper, David Charles, Malvern Park Ashford Kent (GB); Johnson, George Arthur, Gayton Wirral Merseyside (GB); Peter, Donald, Thornton-Hough Wirral Merseyside (GB)
(74) Representative: Mole, Peter Geoffrey

(56) References cited:
- BR-A- 7 604 601
- DE-A- 2 433 703
- DE-A- 2 631 129
- DE-A- 2 653 329
- DE-A- 2 805 767
- DE-A- 2 805 768
- FR-A- 2 269 924
- FR-A- 2 318 265
- FR-A- 2 362 210
- GB-A- 838 240
- GB-A- 1 589 866
- GB-A- 2 269 924
- US-A- 3 355 388
- US-A- 4 026 813
- US-A- 4 115 326
- US-A- 4 115 431
- US-A- 4 132 676
- US-A- 4 142 997
- Whitehouse & Corter: The Proc. of the Scientific Section of the Toilet Goods Assoc. Vol. 18, 1967, pp. 31-37
- Ullmanns Enzyklopädie der technischen Chemie, 3. ed. 1967, vol. 18, p. 344

## Description

This invention relates to deodorant detergent products for use in suppressing human body malodour.

### Background to the invention

It has long been recognised that the development of body malodour is largely due to bacterial action on the products of the sweat glands. Washing the skin with a detergent, for instance in the form of a personal washing detergent bar, removes malodorous products and reduces the concentration of bacteria on the skin. Likewise, washing soiled clothing with a fabric washing detergent product, for instance in the form of a powder or liquid detergent product, removes malodorous products and bacteria derived from the skin.

It has been customary to incorporate germicides into detergent products, particularly those designed for personal washing, in the belief that growth of those skin microflora that contribute to body malodour can be inhibited and the production of malodorous substances suppressed. Germicides are at least partly effective in reducing or retarding the development of body malodour, but they do not completely solve the problem, possibly because there are other causes of malodour development on the skin which are unrelated to the proliferation of bacteria.

### Summary of the invention

It has now been discovered that certain combinations of substances other than germicides, hereinafter referred to as "deordorant compositions", when incorporated into a fabric washing detergent product, can be deposited onto the fabric of a garment washed with the product, so that the fabric of the garment then has the property of reducing body malodour when the garment is subsequently worn or otherwise applied in contact with the skin.

In the course of attempts to characterise this new principle, many hundreds of substances and blends of substances have been screened for evidence of their deodorant activity.

### Definition of the invention

In its widest aspect, the invention provides for the use of a deodorant detergent product for washing fabric to impart to said fabric the ability to suppress human body malodour when the fabric is worn as a garment, the detergent product comprising:
(i) from 5 to 95% by weight if the product is solid, from 5 to 80% by weight if the product is a liquid, of non-soap detergent active compound chosen from anionic, nonionic, cationic, amphoteric and zwitterionic detergent active compounds;
(ii) from 0.05 to 3% by weight of a deodorant composition comprising a combination of perfume materials that depress the partial vapour pressure of morpholine by at least 10% more than that required by Raoult's Law, the deodorant composition having a Deodorant Value, (as measured by the Whitehouse and Carter test modified by the use of a 0 to 5 grading scale, and assessment after 5 hours instead of 24 hours), when incorporated into a personal washing bar at a concentration of 1.5% by weight, of from 0.50 to 3.5; and
(iii) other detergent adjuncts which include a builder and form the balance of the product;
the detergent product having an Odour Reduction Value of at least 0.50, the Odour Reduction Value being defined by the Odour Reduction Value Test, which in turn is based on the Whitehouse and Carter Test with the following modification, namely that assessment of body malodour is performed 5 hours after treatment instead of 24 hours, using a 0 to 5 instead of a 0 to 10 odour grading scale, the Test being carried out following application to shirt fabric during the wash cycle of a laundry process, the body malodour remaining on the shirt fabric being assessed, rather than the malodour of the axilla itself, the fabric washing and odour assessment being conducted as follows:
(a) polyester coat style button through shirts are prewashed in an automatic washing machine using a nonionic detergent fabric washing powder to ensure that the shirts to be used in the Odour Reduction Value Test are all equally clean and free from dressing prior to washing in the detergent product;
(b) the prewashed shirts are line dried and washed again in the automatic washing machine in which the wash liquor contains 0.4% by weight of the fabric washing powder or liquid product under test containing 0.2% by weight of the deodorant composition, the ratio of shirt fabric (dry weight basis) to wash liquor being 40g fabric per litre wash liquor;
(c) the shirts are agitated in the wash liquor for 10 minutes at a temperature of 50°C, then rinsed and spun to a moisture content of about 50% by weight water and finally line dried to a moisture content of not greater than 10% to provide test shirts;
(d) a further batch of the prewashed shirts to serve as control shirts are washed again and then dried under similar conditions, except that the deodorant composition is omitted from the fabric washing powder or liquid product added to the wash liquor;
(e) the shirts are folded and stored overnight in polyethylene bags until required for testing by a panel of male subjects, half the subjects then wearing test shirts treated with the deodorant composition-containing fabric washing powder or liquid product and half wearing control shirts without deodorant composition treatment; and
(f) after 5 hours, the odour of the shirt fabric in the region of the axilla is scored in each case by female assessors, the Odour Reduction Value of the fabric washing powder or liquid product being finally calculated as the difference between the average score for the shirts washed with the test product and the average score for the shirts washed with the control product.

The invention furthermore provides a method for suppressing human body malodour which comprises contacting the skin with a fabric treated with a deodorant detergent product as herein defined.

It is a preferred property of the deodorant detergent product of the invention that it should comprise a deodorant composition which satisfies a deodorancy test when applied to the skin of human subjects. The average amount by which body malodour should be reduced is expressed in terms of the deodorant value of the deodorant composition contained in the detergent product. Products of the invention accordingly preferably comprise a deodorant composition having a deodorant value of from 0.50 to 3.5. Products in which the deodorant composition has a deodorant value of below 0.50 are outside the scope of this invention and are considered to be incapable of reducing body malodour to a significant extent.

### The deodorant value test

In this test the deodorant value of a deodorant composition is measured by assessing its effectiveness, when contained in a standard soap bar at a standard concentration, in reducing body malodour when the standard soap bar is used to wash the axillae (armpits) of a panel of human subjects.

The choice of a soap base is not critical to the performance of the test but as illustrative of the conduct of the test in this respect the procedure followed in the preparation of the base employed in many of the tests referred to later in this specification is included in the description of the test.

Standard soap bars are prepared as follows, all amounts given being by weight.

As a soap base there is used a neutral wet sodium soap containing 63% of total fatty matter of which 82% is tallow fatty acid and 18% is coconut oil fatty acid. To a homogeneous mixture of 9000 parts of this soap base and 340 parts of free coconut oil fatty acid at 80°C are added with mixing, 9.4 parts of a 20% aqueous solution of tetrasodium ethylenediamine tetraacetate, 2.2 parts of a 60% aqueous solution of 1 - hydroxyethane - 1,1 - diphosphonic acid and 7.2 parts of butylated hydroxy toluene (BHT) antioxidant dissolved in a little methylated spirits and the temperature of the mass is raised to 140°C under superatmospheric pressure. The mass is then sprayed at about 30 mm of mercury, to produce a dried soap composition which is collected and extruded at 30°C as noodles of about 12% moisture content.

9,770 parts of the soap noodles thus obtained are mixed at ambient temperature with 150 parts of the deodorant composition to be tested, together with 30 parts of a titanium dioxide opacifier and 50 parts of a colourant suspension. The resulting mixture is milled and plodded in conventional equipment, cut into billets and stamped into bars. The deodorant composition to be tested is therefore present at the standard level of 1.5%. These bars are described as 80/20/5 soap base and consist of 80 parts tallow soap and 20 parts coconut soap, 5 parts of this soap mixture being free fatty acids expressed as coconut oil fatty acid.

Control soap bars are prepared in a similar manner except that the deodorant composition is omitted. In other respects, the control bar should only contain those additives conventionally present in personal washing products and for the purpose in the amount conventionally used in the art. For example, it is permissible as indicated in the foregoing description to include antioxidants in the control bar, but these should be present only in the amount required to stabilise the soap base.

The test is conducted in a manner based on that devised by Whitehouse & Carter as published in "The Proceedings of the Scientific Section of the Toilet Goods Association", No. 18, December 1967, at pages 31 to 37, under the title "Evaluation of Deodorant Toilet Bars".

The test described in that publication was modified in three ways: first, the standard soap bar containing 1.5% by weight of a deodorant composition instead of germicides was employed, secondly, a 0 to 5 instead of a 0 to 10 grading scale was employed as a basis for determining the deodorant value, and thirdly, grading of odour intensity was performed 5 hours instead of 24 hours after treatment. This test is referred to herein as the Deodorant Value test.

Although the invention in its widest aspect provides for the use of deodorant detergent products comprising deodorant compositions having a deodorant value of from 0.50 to 3.5, preferred deodorant detergent products are those comprising deodorant compositions which have a deodorant value of at least 0.60, or 0.70, or 0.80, or 0.90, or 1.00, or 1.20, the higher the minimum value, the more effective is the product as a deodorant detergent product.

### Non-soap detergent active compound

Non-soap detergent active compounds suitable for use in deodorant detergent products of the invention can be non-soap anionic or nonionic or cationic or amphoteric or Zwitterionic in character. Typical non-soap anionic detergent-active compounds include water-soluble salts, particularly the alkali metal, ammonium and alkanolammonium salts, of organic sulphuric reaction products having in their molecular structure an alkyl group containing from about 8 to about 22 carbon atoms and a sulphuric acid or sulphuric acid ester group. (Included in the term "alkyl" is the alkyl portion of acyl groups). Examples of this group of non-soap detergents which can be used are the sodium and potassium alkyl sulphates, especially those obtained by sulphating the higher alcohols (C₈―C₁₈ carbon atoms) produced by reducing the glycerides of tallow or coconut oil; and sodium and potassium alkyl benzene sulphonates, in which the alkyl group contains from about 9 to about 15 carbon atoms in straight chain or branched chain configuration.

Other non-soap anionic detergent-active compounds include the sodium alkyl glycerol ether sulphonates, especially those ethers or higher alcohols derived from tallow and coconut oil; sodium coconut oil fatty acid monoglyceride sulphonates and sulphates; and sodium or potassium salts of alkyl phenol ethylene oxide ether sulphate containing about 1 to about 10 units of ethylene oxide per molecule and wherein the alkyl groups contain about 8 to about 12 carbon atoms.

Other useful non-soap anionic detergent-active compounds include the water-soluble salts of esters of α-sulphonated fatty acids containing from about 6 to 20 carbon atoms in the ester group; water-soluble salts of 2 - acyloxy - alkane - 1 - sulphonic acids containing from about 2 to 9 carbon atoms in the acyl group and from about 9 to about 23 carbon atoms in the alkane moiety; alkyl ether sulphates containing from about 10 to 20 carbon atoms in the alkyl group and from about 1 to 30 moles of ethylene oxide; water-soluble salts of olefin sulphonates containing from about 12 to 24 carbon atoms; and 6-alkyloxy alkane sulphonates containing from about 1 to 3 carbon atoms in the alkyl group and from about 8 to 20 carbon atoms in the alkane moiety.

Preferred water-soluble non-soap anionic detergent-active compounds include linear alkyl benzene sulphonates containing from about 11 to 14 carbon atoms in the alkyl group: the tallow range (C₁₂₋₂₀) alkyl sulphates; the coconunt range alkyl glyceryl sulphonates; and alkyl ether sulphates wherein the alkyl moiety contains from about 14 to 18 carbon atoms and wherein the average degree of ethoxylation varies between 1 and 6.

Specific preferred non-soap anionic detergent-active compounds include: sodium linear C₁₀―C₁₂ alkyl benzene sulphonate; triethanolamine C₁₀―C₁₂ alkyl benzene sulphonate; sodium tallow alkylsulphate; and sodium coconut alkyl glyceryl ether sulphonate; and the sodium salt of a sulphated condensation product of tallow alcohol with from about 3 to about 10 moles of ethylene oxide.

It is to be understood that any of the foregoing anionic detergent-active compounds can be used separately or as mixtures.

Examples of suitable nonionic detergent-active compounds are condensates of linear and branched chain aliphatic alcohols or carboxylic acids of from 8 to 18 carbon atoms with ethylene oxide, for instance a coconut alcohol-ethylene oxide condensate of 6 to 30 moles of ethylene oxide per mole of coconut alcohol; condensates of alkylphenols whose alkyl group contains from 6 to 12 carbon atoms with 5 to 25 moles of ethylene oxide per mole of alkylphenol; condensates of the reaction product of ethylenediamine and propylene oxide with ethylene oxide, the condensates containing from 40 to 80% of polyoxyethylene radicals by weight and having a molecular weight of from 5,000 to 11,000; tertiary amine oxides of structure R₃NO, where one group R is an alkyl group of 8 to 18 carbon atoms and the others are each methyl, ethyl or hydroxyethyl groups, for instance dimethyldodecylamine oxide; tertiary phosphine oxides of structure R₃PO, where one group R is an alkyl group of from 10 to 18 carbon atoms, and the others are each alkyl or hydroxyalkyl groups of 1 to 3 carbon atoms, for instance dimethyldodecylphosphine oxide; and dialkyl sulphoxides of structure R₂SO where one group R is an alkyl group of from 10 to 18 carbon atoms and the other is methyl or ethyl, for instance methyltetradecyl sulphoxide.

Suitable cationic detergent-active compounds are quaternary ammonium salts having an aliphatic radical of from 8 to 18 carbon atoms, for instance cetyltrimethyl ammonium bromide.

Examples of suitable amphoteric detergent-active compounds are derivatives of aliphatic secondary and tertiary amines containing an alkyl group of 8 to 18 carbon atoms and an aliphatic radical substituted by an anionic water-solubilising group, for instance sodium 3-dodecylaminopropionate, sodium 3-dodecylaminopropane sulphonate and sodium N - 2 - hydroxydodecyl - N - methyltaurate.

Suitable zwitterionic detergent-active compounds are derivatives of aliphatic quaternary ammonium, sulphonium and phosphonium compounds having an aliphatic radical of from 8 to 18 carbon atoms and an aliphatic radical substituted by an anionic water-solubilising group, for instance 3 - (N,N - dimethyl - N - hexadecylammonium)propane - 1 - sulphonate betaine, 3 - (dodecylmethyl sulphonium)propane - 1 - sulphonate betaine and 3 - (cetylmethylphosphonium)ethane sulphonate betaine.

In addition to any of the above non-soap detergent-active compounds, soaps can optionally also be present. Soaps are salts of fatty acids and include alkali metal soaps such as the sodium, potassium, ammonium and alkanol ammonium salts of higher fatty acids containing from about 8 to about 24 carbon atoms, and preferably from about 10 to about 20 carbon atoms. Particularly useful are the sodium and potassium and mono-, di- and triethanolamine salts of the mixtures of fatty acids derived from coconut oil and tallow.

Further examples of detergent-active compounds are compounds commonly used as surface-active agents given in the well-known textbooks "Surface Active Agents", Volume 1 by Schwartz and Perry and "Surface Active Agents and Detergents", Volume II by Schwartz, Perry and Berch.

The preferred amount of non-soap detergent-active compound that can be incorporated into deodorant detergent products according to the invention will depend on the nature of the product (i.e. whether it is liquid or solid and whether it comprises only non-soap detergents or both soap and non-soap detergents).

It can be stated generally that the amount of non-soap detergent active compound to be employed for solid products is within the range of from 5 to 95% by weight and for liquid products is within the range of from 5 to 80% by weight.

### The deodorant composition

The essential substances required for the formulation of deodorant compositions that are operative according to the new principle are those that depress the partial vapour pressure of morpholine by at least 10% more than that required by Raoult's Law, as determined by the following test, which is designated "The Morpholine Test".

### The morpholine test

In this test the capacity of a substance to depress the partial vapour pressure of morpholine more than that required by Raoult's Law is measured. Substances that undergo chemical reaction with morpholine are to be regarded as excluded from the test, even though they will generally depress the partial vapour pressure of morpholine by at least the defined amount, since not all such substances are operative according to the new principle. It is to be understood, however, that such substances can be included in the formulation of the deodorant composition, provided that, when included, the composition has the ability to reduce odour intensity by at least 0.50 as herein defined.

### The morpholine test is carried out in the following manner:

Into a sample bottle of capacity 20 ml is introduced morpholine (1g) the bottle fitted with a serum cap and then maintained at 37°C for 30 minutes for equilibrium to be reached. The gas in the headspace of the bottle is analysed by piercing the serum cap with a capillary needle through which nitrogen at 37°C is passed to increase the pressure in the bottle by a standard amount and then allowing the excess pressure to inject a sample from the headspace into gas chromatograph apparatus, which analyses it and provides a chromatographic trace curve with a peak due to morpholine, the area under which is proportional to the amount of morpholine in the sample.

The procedure is repeated under exactly the same conditions using instead of morpholine alone, morpholine (0.25g) and the substance to be tested (1g); and also using the substance (1g) without the morpholine to check whether it gives an interference with the morpholine peak (which is unusual).

The procedure is repeated until reproducible results are obtained. The areas under the morpholine peaks are measured and any necessary correction due to interference by the substance is made.

A suitable apparatus for carrying out the above procedure is a Perkin-Elmer Automatic GC Multifract F40 for Head Space Analysis. Further details of this method are described by Kolb in 'CZ-Chemie-Technik", Volume 1, No. 2, 87―91 (1972) and by Jentzsch et al in "Z.Anal.Chem." *236*, 96―118 (1968).

The measured areas representing the morpholine concentration are proportional to the partial vapour pressure of the morpholine in the bottle headspace. If A is the area under the morpholine peak when only morpholine is tested and A' is the area due to morpholine when a substance is present, the relative lowering of partial vapour pressure of morpholine by the substance is given by 1-A'/A.

According to Raoult's Law, if at a given temperature the partial vapour pressure of morpholine in equilibrium with air above liquid morpholine is p, the partial vapour pressure of p' exerted by morpholine in a homogeneous liquid mixture of morpholine and the substance at the same temperature is $\text{pM/(M+PC)}$, where M and PC are the molar concentrations of morpholine and the substance. Hence, according to Raoult's Law the relative lowering of morpholine partial vapour pressure (p-p')/p, is given by $\text{1-M/(M+PC)}$, which under the circumstances of the test is 87/(87+m/4), where m is the molecular weight of the substance.

The extent to which the behaviour of the mixture departs from Raoult's Law is given by the ratio$\frac{\text{1-A'/A}}{\text{87/(87+m/4)}}$

The above ratio, which will be referred to as the Raoult Variance Ratio, is calculated from the test results. Where a substance is a mixture of compound, a calculated or experimentally determined average molecular weight is used for m. A substance that depresses the partial vapour pressure of morpholine by at leust 10% more than that required by Raoult's Law is one in which the Raoult Variance Ratio is at least 1.1.

Deodorant compositions can be incorporated in deodorant detergent products according to the invention, at a concentration of from from 0.05 to 3%, preferably from 0.1 to 1% by weight.

It is apparent that if less than 0.01% of a deodorant composition is employed, then use of the detergent product is unlikely to provide a significant reduction in body malodour intensity. If more than 10% of a deodorant composition is employed, then use of the detergent product is unlikely to further reduce body malodour intensity beyond that observed at the 10% level.

### Detergent adjuncts

Deodorant detergent products of the invention contain builder and can contain other detergent composition ingredients (adjuncts) for instance sequestrants, soil release agents, antiredeposition agents, superfatting agents such as free long-chain fatty acids, lather boosters such as coconut monoethanolamide; lather controllers; inorganic salts such as sodium and magnesium sulphates; moisturisers; plasticisers and thickeners; opacifiers, colourants, fluorescers, bleaching agents, perfumes, germicides and other deodorant materials such as zinc ricinoleate. The product can also contain water.

The total amount of detergent adjuncts that can be incorporated into the deodorant detergent product according to the invention will normally form the balance of the product after accounting for the deodorant composition and the detergent-active compound.

### Product types and formulations

The deodorant detergent product can be formulated as a solid product, for example in the form of a bar such as a laundry bar, or a powder which can be used for fabric washing. A bar or powder according to the invention comprises from 5 to 95% by weight of the non-soap detergent active compound, and from 0.5 to 3% by weight of the deodorant composition.

Alternatively, the product can take the form of a liquid product for use in fabric washing.

A liquid product according to the invention comprises from 5 to 80% by weight of the non-soap detergent active compound, and from 0.5 to 3% by weight of the deodorant composition.

As a further alternative, the product can take the form of a gelled product for use in fabric washing.

It is understood that the foregoing products are examples of forms which the deodorant detergent product can take: other product forms within the purview of the art are to be included within the scope of monopoly claimed.

The invention is further illustrated by the following examples of detergent product formulations which can be used as a basis for incorporating deodorant compositions to form deodorant detergent products according to the invention.

Examples of the detergent bars containing non-soap detergents which can be employed as a basis for incorporation of a deodorant composition to provide deodorant detergent products according to the invention are as follows:

| Laundry Bar D | % w/w |
|---|---|
| C₆―C₁₆ alkyl benzene sulphonate | 28 |
| Sodium tripolyphosphate | 23 |
| Calcium carbonate | 23.9 |
| Sodium carbonate | 10 |
| Sodium carboxymethylcellulose | 1.42 |
| Water | 6.5 |
| Other ingredients including titanium dioxide, fluorescer, colouring matter and inorganic salts | to 100 |

| Laundry Bar E | % w/w |
|---|---|
| C₆―C₁₆ alkyl benzene sulphonate | 15 |
| Sodium tripolyphosphate | 15 |
| Calcium carbonate | 37 |
| Starch | 7 |
| Sodium carbonate | 10 |
| Sodium carboxymethylcellulose | 0.71 |
| Water | 11 |
| Other ingredients including titanium dioxide, fluorescer, colouring matter and inorganic salts | to 100 |

| Laundry Bar F | % w/w |
|---|---|
| C₆―C₁₆ alkyl benzene sulphonate | 32 |
| Sodium pyrophosphate | 10 |
| Calcium carbonate | 40 |
| Sodium carbonate | 5 |
| Water | 4 |
| Other ingredients including titanium dioxide, fluorescer, colouring matter and inorganic salts | to 100 |

Examples of fabric washing powders which can be employed as a basis for incorporation of a deodorant composition to provide a deodorant detergent product according to the invention are as follows:

| Fabric Washing Powder A | % w/w |
|---|---|
| Sodium C₁₃―C₁₈ alkane sulphonate | 8.0 |
| C₁₆₋₂₀ n-alcohol+25 moles ethylene oxide | 3.4 |
| Sodium soap (containing 4 parts tallow fatty acid to 1 part coconut fatty acid) | 3.4 |
| Pentasodium tripolyphosphate | 37.3 |
| Sodium sulphate | 6.7 |
| Sodium carboxymethylcellulose | 2.0 |
| Ethylene diamine tetra acetic acid | 1.0 |
| Magnesium silicate | 2.0 |
| Fluorescer | 0.3 |
| Waterglass powder (Na₂O:SiO₂=1:3.4) | 5.9 |
| Sodium carbonate | 1.0 |
| Sodium perborate monohydrate | 19.0 |
| Water | 10.0 |

| Fabric Washing Powder B | % w/w |
|---|---|
| C₁₁₋₁₅ n-alcohol+7 moles ethylene oxide | 12 |
| Pentasodium tripolyphosphate | 37.3 |
| Sodium sulphate | 6.7 |
| Sodium carboxymethylcellulose | 2.0 |
| Ethylenediamine tetra acetic acid | 1.0 |
| Magnesium silicate | 2.0 |
| Fluorescer | 0.3 |
| Water glass powder (Na₂O:SiO₂=1:34) | 5.9 |
| Sodium carbonate | 1.0 |
| Sodium perborate monohydrate | 19.0 |
| Water | to 100 |

| Fabric Washing Powder C | % w/w |
|---|---|
| Sodium dodecyl benzene sulphonate | 14 |
| Sodium tripolyphosphate | 40 |
| Sodium sulphate | 2 |
| Sodium carboxymethylcellulose | 1 |
| Ethylene diamine tetra acetic acid | 1 |
| Magnesium silicate | 2 |
| Fluorescer | 0.3 |
| Water glass powder (Na₂O:SiO₂=1:3.4) | 5.9 |
| Sodium carbonate | 1.0 |
| Sodium perborate monohydrate | 25 |
| Water | to 100 |

| Fabric Washing Powder D | % w/w |
|---|---|
| Sodium dodecyl benzene sulphonate | 8 |
| Tallow alcohol sulphate | 6 |
| Sodium tripolyphosphate | 40 |
| Sodium sulphate | 2 |
| Sodium carboxymethylcellulose | 1 |
| Ethylene diamine tetra acetic acid | 1 |
| Magnesium silicate | 2 |
| Fluorescer | 0.3 |
| Water glass powder (Na₂O:SiO₂=1:3.4) | 5.9 |
| Sodium carbonate | 1.0 |
| Sodium perborate monohydrate | 25 |
| Water | to 100 |

| Fabric Washing Powder E | % w/w |
|---|---|
| Sodium dodecyl benzene sulphonate | 15 |
| Tallow alcohol 18 $\overline{\text{EO}}$ | 3 |
| Tallow alcohol 12 $\overline{\text{EO}}$ | 3 |
| Sodium stearate (soap) | 6 |
| Sodium tripolyphosphate | 40 |
| Sodium silicate | 5 |
| Sodium carboxymethylcellulose | 2 |
| Fluorescer | 0.2 |
| EDTA | 0.2 |
| Enzyme | 0.66 |
| Sodium sulphate | 14 |
| Water | to 100 |

| Fabric Washing Powder F | % w/w |
|---|---|
| Sodium dodecyl benzene sulphonate | 15 |
| Tallow alcohol 18 $\overline{\text{EO}}$ | 2.5 |
| Tallow alcohol 12 $\overline{\text{EO}}$ | 2.5 |
| Sodium tripolyphosphate | 30 |
| Sodium silicate | 5 |
| Sodium sulphate | 25 |
| Enzymes | 0.66 |
| EDTA | 0.2 |
| Fluorescer | 0.2 |
| Sodium carboxymethylcellulose | 2 |
| Water | to 100 |

An example of fabric washing liquid which can be employed as a basis for incorporation of a deodorant composition to provide a deodorant detergent product according to the invention is as follows:

| Fabric Washing Liquid B | % w/w |
|---|---|
| Alkyl C₁₂ to C₁₅ alcohol 9 EO | 8 |
| Sodium xylene sulphonate | 2 |
| Sodium pyrophosphate | 2.8 |
| Potassium pyrophosphate | 22 |
| Sodium silicate | 3 |
| Sodium carboxymethylcellulose | 0.38 |
| Fluorescer | 0.1 |
| Water | to 100 |

### Process for preparing deodorant detergent products

The process for preparing deodorant detergent products thereby employing a deodorant composition as a means for inhibiting body malodour development comprises mixing non-soap detergent-active compounds, detergent adjuncts, if present, and a deodorant composition to provide a deodorant detergent product, the deodorant composition having a deodorant value of at least 0.50. The selection of non-soap detergent active compounds and detergent adjuncts and their respective amounts employed in the process of the invention will depend upon the nature of the required detergent product (e.g. solid or liquid) and the purpose for which it is required (i.e. for fabric washing).

Usually it is convenient to add the deodorant composition to the detergent product at a stage towards the end of its manufacture so that loss of any volatile ingredients such as may occur during a heating step is minimised.

It is furthermore usual to incorporate the deodorant composition in such a manner that it is thoroughly mixed with the other ingredients and is uniformly distributed throughout the detergent product. It is however also possible, particularly with solid products such as marbled laundry bars and specked or spotted solid or liquid products, where the deodorant composition can be encapsulated to delay its subsequent release, to provide detergent products where the deodorant composition is not uniformly and homogeneously mixed with the other ingredients of the detergent product, and is concentrated in the marble bands of the specked or spotted parts of such products.

### Method of using the deodorant detergent product

The deodorant detergent product of the invention is to be employed particularly for suppressing human body malodour, by laundering garments which are subsequently to be worn in contact with the skin. The deodorant detergent product is particularly effective when applied by this modes to the regions of the human skin where apocrine sweat glands are most abundant, notably in the groin, axilla, anal and genital regions and in the aureola of the nipple.

In use, the deodorant detergent fabric washing product can, for example, be applied to a garment according to conventional laundering procedures involving water washing, rinsing and drying. It is apparent that sufficient of the deodorant composition is delivered to and remains on the fabric of laundered garments subsequently to enable the wearer to benefit from its deodorising effect by reduction of body malodour.

### Specific examples of the invention

The invention is illustrated by the following Examples of detergent products.

It should be noted that each of these products was evaluated in a manner similar to that described for the Deodorant Value Test referred to hereinbefore. However, in view of the fact that testing the products involved the assessment of body malodour after wearing a shirt which had been washed with such a product, (instead of by washing the axillae with a standard soap bar containing the deodorant composition previously described as the Deodorant Value Test) the effectiveness of each product was expressed in terms of "odour reduction value".

It should be recognised that if an odour reduction value of less than 0.50 is recorded following use of products of the invention, it is indicative that insufficient deodorant composition has been transferred to the skin of the axilla, rather than evidence that the product itself contains insufficient of a deodorant composition as herein defined.

### Example 1

In this Example, the deodorant effectiveness of a non-soap detergent (NSD) fabric washing powder according to the invention was evaluated in terms of its odour reduction value in the following manner:

Polyester cotton coat style button through shirts were first prewashed in an automatic washing machine using a nonionic detergent fabric washing powder. This was to ensure that the shirts to be used in the test were all equally clean and free from dressing prior to washing in the deodorant fabric washing product.

The washed shirts were line dried and then washed again in the automatic washing machine. The test deodorant NSD fabric washing product was then added to the wash liquor at a concentration of 0.4% by weight of the liquor. The ratio of shirt fabric (dry weight basis) to wash liquor was 40g fabric per litre wash liquor.

The shirts were agitated in the wash liquor for 10 minutes at a temperature of 50°C, then rinsed and spun to a moisture content of about 50% water and finally line dried to a moisture content of not greater than 10%.

A further batch of prewashed shirts which were to serve as control shirts were washed again and then dried under similar conditions except that deodorant composition was omitted from the fabric washing product added to the wash liquor.

The shirts were folded and stored overnight in polyethylene bags until required for testing by a panel of male subjects and assessing for odour by a panel of female assessors.

The above procedure was repeated on four consecutive days without prewashing, half of the subjects wearing shirts treated with the deodorant composition-containing detergent product and half wearing control shirts without deodorant composition treatment.

Body odour transferred to the shirts in the region of the axillae was assessed by the trained female assessors in the manner described in the Deodorant Value Test, the odour of the shirt fabric being scored in each case rather than the axillae of the panel subjects and the results expressed as odour reduction value.

The formulation of the fabric washing product was that described hereinbefore as Fabric Washing Powder C.

Test fabric washing product was prepared by the addition of 0.2 parts of a deodorant composition to 99.8 parts of the above formulation.

The formulation of Deodorant Composition 1 was as follows:

| Deodorant Composition 1 | Parts |
|---|---|
| Amber AB 358 | 3.0 |
| iso-Amyl salicylate | 5.0 |
| Benzyl salicylate | 4.0 |
| Bergamot AB 430 | 15.0 |
| o-t-Butylcyclohexyl acetate | 0.5 |
| Cedar atlas oil | 5.0 |
| Citronellol | 7.0 |
| Citronella oil | 16.1 |
| Citronellyloxyacetaldehyde | 0.5 |
| Geranium base 76 | 4.0 |
| 1,3,4,6,7,8-Hexahydro-4,6,6,7,8,8-hexamethyl cyclopenta-γ-2-benzopyran | 10.0 |
| Hexyl aldone | 0.7 |
| Jasmin AB 284 | 12.0 |
| LRG 201 | 5.0 |
| Nonanolide-1:4 | 0.2 |
| Opoponax resinoid | 1.7 |
| Orange oil sweet | 8.0 |
| 10-Undecen-1-al | 0.30 |
| Vetyvert oil | 2.0 |
| | $\overline{\text{100.00}}$ |

The Results of Odour Reduction Value Test 1 (using NSD Powder C and Deodorant Composition 1) were as follows:

| | Control powder | Test powder |
|---|---|---|
| Average scores | 2.94 | 1.97 |
| Odour reduction value | | 0.97 |

### Example 2

The procedure described in Example 1 was repeated using NSD Fabric Washing Powder B as hereinbefore described instead of Fabric Washing Powder C and using Deodorant Composition 4.

The formulation of Deodorant Composition 4 was as follows:

| Deodorant Composition 4 | Parts |
|---|---|
| Bergamot AB 430 | 8.00 |
| p-t-Butylcyclohexyl acetate | 4.30 |
| Citronella oil | 6.0 |
| Diethyl phthalate | 8.25 |
| Ethyl vanillin | 0.20 |
| iso-Eugenol | 5.00 |
| Green Herbal AB 502 | 15.00 |
| 2-n-Heptylcyclopentanone | 0.50 |
| Indole | 1.50 |
| Inonyl formate | 5.00 |
| LRG 201 | 1.25 |
| α-iso-Methyl ionone | 5.00 |
| β-Naphthol methylether | 7.50 |
| Nonanediol-1:3-diacetate | 4.00 |
| Patchouli oil | 7.00 |
| Phenylethyl phenyl acetate | 5.00 |
| Rosenta AB 380 | 6.00 |
| Sandalone | 4.00 |
| Tetrahydro muguol | 6.00 |
| γ-Undecalactone | 0.50 |
| | $\overline{\text{100.00}}$ |

The Results of Odour Reduction Value Test 2 (using NSD Powder B and Deodorant Composition 4) were as follows:

| | Control powder | Test powder |
|---|---|---|
| Average scores | 2.69 | 1.62 |
| Odour reduction value | | 1.07 |

### Example 3

The procedure described in Example 1 was repeated using NSD Fabric Washing Liquid B as hereinbefore described instead of Fabric Washing Powder C and using Deodorant Composition 3.

The formulation of Deodorant Composition 3 was as follows:

| Deodorant Composition 3 | Parts |
|---|---|
| p-t-Amylcyclohexanone | 5.00 |
| Benzoin Siam resinoid | 5.00 |
| Bergamot AB 430 | 15.00 |
| Coumarin | 4.00 |
| Diethyl phthalate | 4.35 |
| Geranium oil | 5.00 |
| Hercolyn D | 12.25 |
| 1,3,4,6,7,8-Hexahydro-4,6,6,7,8,8-hexamethyl cyclopenta-γ-2-benzopyran | 3.00 |
| Lavandin oil | 10.00 |
| α-iso-Methyl ionone | 12.00 |
| Mousse de chene yugo | 1.25 |
| Musk ambrette | 3.00 |
| Pimento leaf oil | 10.00 |
| Rosenta AB 380 | 10.00 |
| Rose-D-oxide | 0.15 |
| | $\overline{\text{100.00}}$ |

The results of Odour Reduction Value Test 3 (using NSD liquid B and Deodorant Composition 3) were as follows:

| | Control liquid | Test liquid |
|---|---|---|
| Average scores | 2.64 | 2.14 |
| Odour reduction value | | 0.50 |

### Example 4

The procedure described in Example 1 was repeated using NSD Fabric Washing Powder D as hereinbefore described instead of Fabric Washing Powder C and using Deodorant Composition 2.

The formulation of Deodorant Composition 2 was as follows:

| Deodorant Composition 2 | Parts |
|---|---|
| 6-Acetyl-1,1,3,4,4,6-hexamethyl-tetrahydro naphthalate | 3.00 |
| Bergamot base 37 | 20.00 |
| Carvacrol | 3.50 |
| Citronellyl acetate | 5.00 |
| Dipropylene glycol | 4.75 |
| Geranyl nitrile | 1.50 |
| Indole | 1.00 |
| Lemongrass oil | 3.00 |
| Lime AB 402 | 10.00 |
| Lavandin oil | 4.00 |
| 1-Menthol | 8.00 |
| 3a-Methyl-dodecahydro-6,6,9a-trimethyl naphtho-2(2,1-b)-furan | 0.25 |
| β-Methyl naphthyl ketone | 5.00 |
| β-Naphthol methyl ether | 9.00 |
| Neroli base 78 | 6.00 |
| Pomeransol AB 314 | 6.00 |
| Petitgrain oil (terpeneless) | 4.00 |
| Orange oil sweet | 5.00 |
| Thyme oil red | 1.00 |
| | $\overline{\text{100.00}}$ |

The results of Odour Reduction Value Test 4 (using NSD powder D and Deodorant Composition 2) were as follows:

| | Control powder | Test powder |
|---|---|---|
| Average scores | 2.70 | 1.76 |
| Odour reduction value | | 0.94 |

### Example 5

The procedure described in Example 1 was repeated using NSD Fabric Washing Powder F as hereinbefore described instead of Fabric Washing Powder C and using Deodorant Composition 3 as described in Example 3.

The results of Odour Reduction Test 5 (using NSD powder F plus Deodorant Composition 3) were as follows:

| | Control powder | Test powder |
|---|---|---|
| Average scores | 2.76 | 1.70 |
| Odour reduction value | | 1.06 |

Further examples of Deodorant Compositions as described herein are as follows:

| Deodorant Composition 5 | Parts |
|---|---|
| 6-Acetyl-1,1,3:4,4,6-Hexamethyl tetrahydro naphthalate | 2.5 |
| p-t-Amylcyclohexanone | 0.06 |
| Benzyl salicylate | 15.0 |
| Bergamot AB 430 | 15.0 |
| Cinnamic alcohol | 5.0 |
| Diethyl phthalate | 8.04 |
| Dimethyl benzyl carbinyl acetate | 2.5 |
| Dimyrcetol | 16.0 |
| Dipropylene glycol | 14.25 |
| Geraniol | 5.0 |
| Isobutyl phenyl acetate | 5.0 |
| 3a-Methyl-dodecahydro-6,6,9a-trimethyl-naphtho-2(2,1-b) furan | 0.75 |
| Methyl salicylate | 0.5 |
| Mousse de Chene Yugo | 6.0 |
| Nonanolide-1:4 | 0.2 |
| Pelargene | 4.0 |
| Trichloromethyl phenyl carbinyl acetate | 0.2 |

| Deodorant Composition 6 | Parts |
|---|---|
| Benzyl propionate | 4.0 |
| Bergamot oil | 15.0 |
| o-t-Butylcyclohexyl acetate | 2.0 |
| p-t-Butyl-α-methyl hydrocinnamic aldehyde | 15.0 |
| Clove leaf oil | 10.0 |
| Diethyl phthalate | 9.25 |
| Dimethyl benzyl carbinyl acetate | 5.0 |
| Inonyl acetate | 10.0 |
| iso-Butyl benzoate | 5.0 |
| LRG-201 | 1.25 |
| 3a-Methyl-dodecahydro-6,6,9a-trimethyl-naphtho-2 (2,1-b)-furan | 0.5 |
| Neroli oil | 3.0 |
| Petitgrain oil | 10.0 |
| Phenyl ethyl alcohol | 10.0 |

### Deodorant value of deodorant composition 1 to 6

The deodorant value of each of the deodorant compositions illustrated in the foregoing Examples was determined by the Deodorant Value Test as described hereinbefore using the standard 80/20/5 soap base. The results were as follows:

| Deodorant composition | Average scores | | Deodorant value |
|---|---|---|---|
| | Control bar | Test bar | |
| 1 | 3.46 | 2.93 | 0.53 |
| 2 | 3.34 | 2.73 | 0.61 |
| 3 | 3.04 | 2.47 | 0.57 |
| 4 | 3.25 | 2.10 | 1.15 |
| 5 | 3.30 | 2.70 | 0.60 |
| 6 | 3.25 | 2.33 | 0.92 |

It can be seen from the above results that each of the Deodoran Compositions 1 to 6 had a deodorant value which was greater than 0.50, which defines the minimum deodorant value of a deodorant composition suitable for use in the deodorant detergent products of the invention.

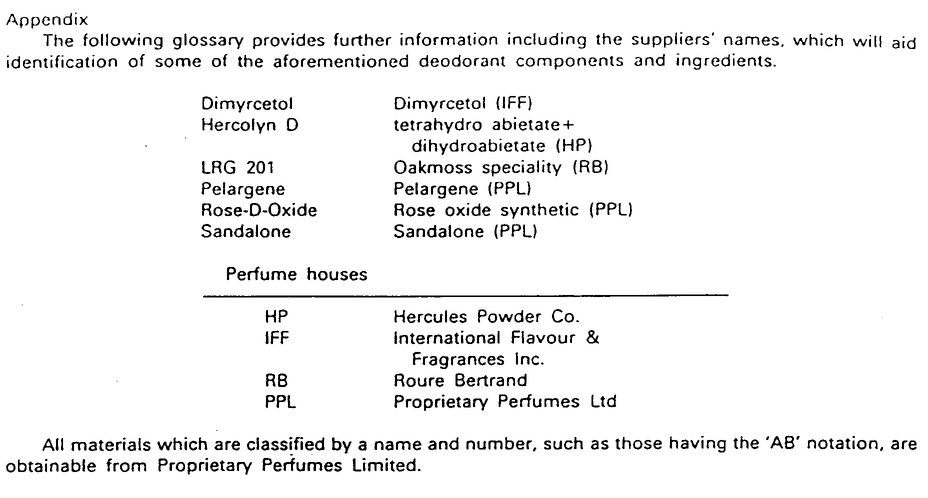

## Claims

1. The use of a deodorant detergent product for washing fabric to impart to said fabric the ability to suppress human body malodour when the fabric is worn as a garment, the detergent product comprising:
(i) from 5 to 95% by weight if the product is solid, from 5 to 80% by weight if the product is a liquid, of non-soap detergent active compound chosen from anionic, nonionic, cationic, amphoteric and zwitterionic detergent active compounds;
(ii) from 0.05 to 3% by weight of a deodorant composition comprising a combination of perfume materials that depress the partial vapour pressure of morpholine by at least 10% more than that required by Raoult's Law, the deodorant composition having a Deodorant Value, (as measured by the Whitehouse and Carter test modified by the use of a 0 to 5 grading scale, and assessment after 5 hours instead of 24 hours), when incorporated into a personal washing bar at a concentration of 1.5% by weight, of from 0.50 to 3.5; and
(iii) other detergent adjuncts which include a builder and form the balance of the product;
the detergent product having an Odour Reduction Value of at least 0.50, the Odour Reduction Value being defined by the Odour Reduction Value Test, which in turn is based on the Whitehouse and Carter Test with the following modification, namely that assessment of body malodour is performed 5 hours after treatment instead of 24 hours, using a 0 to 5 instead of a 0 to 10 odour grading scale, the Test being carried out following application to shirt fabric during the wash cycle of a laundry process, the body malodour remaining on the shirt fabric being assessed, rather than the malodour of the axilla itself, the fabric washing and odour assessment being conducted as follows:
(a) polyester coat style button through shirts are prewashed in an automatic washing machine using a nonionic detergent fabric washing powder to ensure that the shirts to be used in the Odour Reduction Value Test are all equally clean and free from dressing prior to washing in the detergent product;
(b) the prewashed shirts are line dried and washed again in the automatic washing machine in which the wash liquor contains 0.4% by weight of the fabric washing powder or liquid product under test containing 0.2% by weight of the deodorant composition, the ratio of shirt fabric (dry weight basis) to wash liquor being 40g fabric per litre wash liquor;
(c) the shirts are agitated in the wash liquor for 10 minutes at a temperature of 50°C, then rinsed and spun to a moisture content of about 50% by weight water and finally line dried to a moisture content of not greater than 10% to provide test shirts;
(d) a further batch of the prewashed shirts to serve as control shirts are washed again and then dried under similar conditions, except that the deodorant composition is omitted from the fabric washing powder or liquid product added to the wash liquor;
(e) the shirts are folded and stored overnight in polyethylene bags until required for testing by a panel of male subjects, half the subjects then wearing test shirts treated with the deodorant composition-containing fabric washing powder or liquid product and half wearing control shirts without deodorant composition treatment; and
(f) after 5 hours, the odour of the shirt fabric in the region of the axilla is scored in each case by female assessors, the Odour Reduction Value of the fabric washing powder or liquid product being finally calculated as the difference between the average score for the shirts washed with the test product and the average score for the shirts washed with the control product.

2. The use of a detergent product according to Claim 1, in which the non-soap detergent active compound comprises an anionic detergent-active compound chosen from:
(a) sodium and potassium alkyl sulphates obtained by sulphating the C₈―C₁₈ alcohols produced by reducing the glycerides of tallow or coconut oil;
(b) sodium and potassium alkyl benzene sulphonates in which the alkyl group contains from 9 to 15 carbon atoms in straight chain or branched chain configuration;
(c) sodium alkyl glycerol ether sulphonates;
(d) sodium coconut oil fatty acid monoglyceride sulphonates and sulphates;
(e) sodium or potassium salts of alkyl phenol ethylene oxide ether sulphate containing from 1 to 10 units of ethylene oxide per molecule and wherein the alkyl groups contain from 8 to 12 carbon atoms;
(f) water-soluble salts of esters of -sulphonated fatty acids containing from 6 to 20 carbon atoms in the ester group;
(g) water-soluble salts of 2-acyloxy-alkane-1-sulphonic acids containing from 2 to 9 carbon atoms in the acyl group and from 9 to 23 carbon atoms in the alkane moiety;
(h) alkyl ether sulphates containing from 10 to 20 carbon atoms in the alkyl group and from 1 to 30 moles of ethylene oxide;
(i) water-soluble salts of olefin sulphonates containing from 12 to 24 carbon atoms; and
(j) 6-alkyloxyalkanesulphonates containing from 1 to 3 carbon atoms in the alkyl group and from 8 to 20 carbon atoms in the alkane moiety.

3. The use of a detergent product according to Claim 1, in which the non-soap detergent active compound comprises an anionic detergent-active compound chosen from:
(a) linear alkyl benzene sulphonates containing from 11 to 14 carbon atoms in the alkyl group;
(b) tallow (C₁₂―C₂₀) alkyl sulphates;
(c) coconut alkyl glyceryl sulphonates; and
(d) alkyl ether sulphates wherein the alkyl moiety contains from 14 to 18 carbon atoms and wherein the average degree of ethoxylation is from 1 to 6.

4. The use of a detergent product according to Claim 1, in which the non-soap detergent-active compound comprises an anionic detergent-active compound chosen from:
(a) sodium linear C₁₀―C₁₂ alkyl benzene sulphonate;
(b) triethanolamine C₁₀―C₁₂ alkyl benzene sulphonate;
(c) sodium tallow alkyl sulphate;
(d) sodium coconut alkyl glyceryl ether sulphonate; and
(e) sodium salt of a sulphonated condensation product of tallow alcohol with from 3 to 10 moles of ethylene oxide.

5. The use of a detergent product according to any preceding claim, in which the non-soap detergent active compound comprises a nonionic detergent-active compound chosen from:
(a) condensates of linear and branched chain aliphatic alcohols or carboxylic acids of from 8 to 18 carbon atoms, with ethylene oxide;
(b) condensates of alkylphenols whose alkyl group contains from 6 to 12 carbon atoms with 5 to 25 moles of ethylene oxide per mole of alkylphenol;
(c) condensates of the reaction product of ethylene diamine and propylene oxide with ethylene oxide, the condensates containing from 40 to 80% of polyoxyethylene radicals by weight and having a molecular weight of from 5000 to 11000;
(d) tertiary amine oxides of structure R₃NO, where one group R is an alkyl group of 8 to 18 carbon atoms and the others are each methyl, ethyl or hydroxyethyl groups;
(e) tertiary phosphine oxides of structure R₃PO, where one group R is an alkyl group of from 10 to 18 carbon atoms and the others are each alkyl or hydroxyalkyl groups of from 1 to 3 carbon atoms; and
(f) dialkyl sulphoxides of structure R₂SO, where one group R is an alkyl group of from 10 to 18 carbon atoms and the other is methyl or ethyl.

6. The use of a detergent product according to any preceding claim, in which the deodorant composition forms from 0.1 to 1% by weight of the product.

7. The use of a detergent product according to any preceding claim, in which the builder is chosen from sodium tripolyphosphate, sodium pyrophosphate and potassium pyrophosphate.

8. The use of a detergent product according to any preceding claim, in which the other detergent adjuncts comprise a bleaching agent.

9. The use of a detergent product according to claim 8, in which the bleaching agent comprises perborate.

10. The use of a detergent product according to any preceding claim, in which the other detergent adjuncts comprise an enzyme.

11. The use of a detergent product according to any preceding claim in which the other detergent adjuncts comprise a fluorescer.

12. The use of a detergent product according to any preceding claim, which is a fabric washing powder.

13. The use of a detergent product according to any of claims 1 to 11, which is a fabric washing liquid.

## Patentansprüche

1. Verwendung einer desodorierenden Reinigungsmittelzusammensetzung zum Waschen von Textilien um den Textilien die Fähigkeit zu verleihen, unangenehmen, menschlichen Körpergeruch zu unterdrücken, wenn die Textilien als Kleidungsstücke getragen werden, wobei die Reinigungsmittelzusammensetzung umfaßt:
(i) bei festen Zusammensetzungen 5 bis 95 Gew.-%, bei flüssigen Zusammensetzungen 5 bis 80 Gew.-% einer nicht-seifenartigen, Detergens-aktiven Verbindung, ausgewählt unter anionischen, nichtionischen, kationischen, amphoteren und zwitterionischen Detergens-aktiven Verbindungen;
(ii) 0,05 bis 3 Gew.-% einer desodorierenden Zusammensetzung enthaltend eine Kombination von Riechstoffmaterialien, die den partiellen Dampfdruck von Morpholin um mindestens 10 % mehr als durch das Raoultsche Gesetz verlangt, herabdrücken, wobei die desodorierende Zusammensetzung einen Desodorierungswert (gemessen nach dem Whitehouse- und Carter-Test, modifiziert durch die Verwendung einer 0 bis 5 Klassifizierungsskala und Beurteilung nach 5 Stunden anstelle von 24 Stunden) von 0,50 bis 3,5 besitzt, wenn sie einverleibt ist in ein persönliches Waschstück in einer Konzentration von 1,5 Gew.-% und
iii) andere Detergens-Zusätze enthaltend einen Builder als Rest der Zusammensetzung.
wobei die Reinigungsmittelzusammensetzung einen Geruchsverminderungswert von mindestens 0,50 aufweist, der definiert ist durch den Geruchsverminderungswert-Test, der seinerseits basiert auf dem Whitehouse- und Carter-Test mit der Modifikation, daß die Prüfung des unangenehmen Körpergeruchs durchgeführt wird 5 Stunden nach der Behandlung anstelle von 24 Stunden unter Verwendung einer 0 bis 5 anstelle von 0 bis 10 Geruchs-Klassifizierungsskala, wobei der Test ausgeführt wird nach Anwendung an einem Hemdenstoff während des Waschzyklus eines Waschverfahrens und der unangenehme Körpergeruch, der auf dem Hemdenstoff verbleibt, geprüft wird anstelle des unangenehmen Geruchs der Achselhöhle selbst, wobei das Waschen des Stoffes und die Geruchsprüfung wie folgt ausgeführt werden:
(a) Polyester, Mantelstil, durchgeknöpfte Hemden werden vorgewaschen in einer automatischen Waschmaschine unter Verwendung eines Textilwaschpulvers mit nicht-ionischem Detergens, um sicherzustellen, daß die Hemden, die in dem Geruchsverminderungswert-Test benutzt werden sollen, alle gleichmäßig sauber und frei von Appretur sind vor dem Waschen in der Reinigungsmittelzusammensetzung;
(b) die vorgewaschenen Hemden werden an der Leine getrocknet und wieder gewaschen in einer automatischen Waschmaschine, in welcher die Waschflüssigkeit 0,4 Gew.-% des Textilwaschpulvers oder flüssigen Testprodukts enthaltend 0,2 Gew.-% der Desodorierungs-Zusammensetzung enthält, wobei das Verhältnis von Hemdenstoff (Trockengewichtsbasis) zu Waschflüssigkeit 40 g Stoff je Liter Waschflüssigkeit beträgt;
(c) die Hemden werden in der Waschflüssigkeit bewegt während 10 Minuten bei einer Temperatur von 50 °C, dann gespült und geschleudert auf einen Feuchtigkeitsgehalt von etwa 50 Gew.-% Wasser und schließlich auf der Leine getrocknet, auf einen Feuchtigkeitsgehalt von nicht größer als 10 % zur Bereitstellung der Testhemden;
(d) eine weitere Teilmenge der vorgewaschenen Hemden, die als Kontrollhemden dienen sollen, werden wieder gewaschen und dann getrocknet unter ähnlichen Bedingungen, mit der Ausnahme, daß die desodorierende Zusammensetzung aus dem Textilwaschpulver oder dem flüssigen Produkt, welches der Waschflüssigkeit zugegeben wird, weggelassen wird;
(e) die Hemden werden gefaltet und über Nacht gelagert in Polyethylenbeuteln bis sie benötigt werden für den Test durch eine Testgruppe männlicher Personen, wobei die Hälfte der Personen dann Testhemden trägt, die behandelt wurden mit dem Textilwaschpulver oder dem flüssigen Produkt enthaltend die desodorierende Zusammensetzung, und die Hälfte der Personen die Kontrollhemden trägt ohne Behandlung mit der desodorierenden Zusammensetzung; und
(f) nach 5 Stunden der Geruch des Hemdenstoffs im Bereich der Achselhöhlen in jedem Fall geprüft wird durch weibliche Prüfer, und der Geruchsreduktionswert des Textilwaschpulvers oder flüssigen Produkts schließlich berechnet wird als Differenz zwischen der Durchschnittswertung für die Hemden, die mit dem Testprodukt gewaschen wurden und dem Durchschnittswert für die Hemden, die mit dem Kontrollprodukt gewaschen wurden.

2. Verwendung einer Reinigungsmittelzusammensetzung gemäß Anspruch 1, in welcher die nicht-seifenartige, Detergens-aktive Verbindung eine anionische Detergens-aktive Verbindung umfaßt, ausgewählt unter
(a) Natrium- und Kalium-Alkylsulfaten, erhalten durch Sulfatierung von C₈-C₁₈-Alkoholen, hergestellt durch Reduktion der Glyceride von Talg oder Kokosnußöl;
(b) Natrium- oder Kalium-Alkylbenzolsulfonaten, in welchen die Alkylgruppe 9 bis 15 Kohlenstoffatome in gerader oder verzweigter Kette enthält;
(c) Natriumalkylglycerinethersulfonaten;
(d) Natrium-Kokosnußölfettsäure-Monoglyceridsulfonaten und -sulfaten;
(e) Natrium- oder Kaliumsalzen von Alkylphenol-Ethylenoxid-Ethersulfat enthaltend 1 bis 10 Ethylenoxideinheiten je Molekül, wobei die Alkylgruppen 8 bis 12 Kohlenstoffatome enthalten;
(f) wasserlöslichen Salzen von Estern von alpha-sulfonierten Fettsäuren enthaltend 6 bis 10 Kohlenstoffatome in der Estergruppe;
(g) wasserlöslichen Salzen von 2-Acyloxy-Alkan-1-Sulfonsäuren enthaltend 2 bis 9 Kohlenstoffatome in der Acylgruppe und 9 bis 23 Kohlenstoffatome im Alkanteil;
(h) Alkylethersulfate enthaltend 10 bis 20 Kohlenstoffatome in der Alkylgruppe und 1 bis 30 Mol Ethylenoxid;
(i) wasserlösliche Salze von Olefinsulfonaten enthaltend 12 bis 24 Kohlenstoffatome; und
(j) 6-Alkyloxyalkansulfonate enthaltend 1 bis 3 Kohlenstoffatome in der Alkylgruppe und 8 bis 20 Kohlenstoffatome im Alkanteil.

3. Verwendung einer Reinigungsmittelzusammensetzung gemäß Anspruch 1, worin die nicht-seifenartige, Detergens-aktive Verbindung eine anionische Detergens-aktive Verbindung umfaßt, ausgewählt unter:
(a) lineare Alkylbenzolsulfonate enthaltend 11 bis 14 Kohlenstoffatome in der Alkylgruppe;
(b) Talg(C₁₂-C₂₀)-Alkylsulfate;
(c) Kokosnußalkylglycerylsulfonate; und
(d) Alkylethersulfate, worin der Alkylteil 14 bis 18 Kohlenstoffatome enthält und der durchschnittliche Ethoxylierungsgrad 1 bis 6 beträgt.

4. Verwendung einer Reinigungsmittelzusammensetzung gemäß Anspruch 1, worin die nicht-seifenartige, Detergens-aktive Verbindung eine anionische Detergens-aktive Verbindung umfaßt, ausgewählt unter:
(a) Natrium-linearem C₁₀-C₁₂-Alkyl-Benzolsulfonat;
(b) Triethanolamin C₁₀-C₁₂-Alkyl-Benzolsulfonat;
(c) Natrium-Talgalkylsulfat;
(d) Natrium-Kokosnußalkylglycerylethersulfonat; und
(e) Natrium-Salz eines sulfonierten Kondensationsproduktes von Talgalkohol mit 3 bis 10 Mol Ethylenoxid.

5. Verwendung einer Reinigungsmittelzusammensetzung gemäß einem der vorgehenden Ansprüche, in welcher die nicht-seifenartige, Detergens-aktive Verbindung eine nicht-ionische Detergens-aktive Verbindung umfaßt, ausgewählt unter
(a) Kondensate von linearen und verzweigtkettigen, aliphatischen Alkoholen oder Carbonsäuren mit 8 bis 18 Kohlenstoffatomen mit Ethylenoxid;
(b) Kondensate von Alkylphenolen, deren Alkylgruppe 6 bis 12 Kohlenstoffatome enthält mit 5 bis 25 Mol Ethylenoxid je Mol Alkylphenol;
(c) Kondensate des Reaktionsprodukts von Ethylendiamin und Propylenoxid mit Ethylenoxid, wobei die Kondensate 40 bis 80 Gew.-% von Polyoxyethylenresten enthalten und ein Molekulargewicht von 5.000 bis 11.000 besitzen;
(d) tertiäre Aminoxide der Struktur R₃NO, wobei eine Gruppe R eine Alkylgruppe mit 8 bis 18 Kohlenstoffatomen ist und die anderen jeweils Methyl, Ethyl oder Hydroxyethylgruppen sind;
(e) tertiäre Phosphinoxide der Struktur R₃PO, worin eine Gruppe R eine Alkylgruppe mit 10 bis 18 Kohlenstoffatomen und die anderen jeweils Alkyl- oder Hydroxyalkylgruppen mit 1 bis 3 Kohlenstoffatomen sind; und
(f) Dialkylsulfoxide der Struktur R₂SO, worin eine Gruppe R eine Alkylgruppe mit 10 bis 18 Kohlenstoffatomen und die andere Methyl oder Ethyl ist.

6. Verwendung einer Reinigungsmittelzusammensetzung gemäß einem der vorgehenden Ansprüche, worin die desodorierende Zusammensetzung 0,1 bis 1 Gew.-% der Zusammensetzung ausmacht.

7. Verwendung einer Reinigungsmittelzusammensetzung gemäß einem der vorgehenden Ansprüche, worin der Builder ausgewählt ist unter Natriumtripolyphosphat, Natriumpyrophosphat und Kaliumpyrophosphat.

8. Verwendung einer Reinigungsmittelzusammensetzung gemäß einem der vorgehenden Ansprüche, worin der andere Detergens-Zusatz ein Bleichmittel umfaßt.

9. Verwendung einer Reinigungsmittelzusammensetzung gemäß Anspruch 8 , worin das Bleichmittel Perborat umfaßt.

10. Verwendung einer Reinigungsmittelzusammensetzung gemäß einem der vorgehenden Ansprüche, worin der andere Detergens-Zusatz ein Enzym umfaßt.

11. Verwendung einer Reinigungsmittelzusammensetzung gemäß einem der vorgehenden Ansprüche, worin der andere Detergens-Zusatz ein Fluoresziermittel umfaßt.

12. Verwendung einer Reinigungsmittelzusammensetzung gemäß einem der vorgehenden Ansprüche, welche ein Textilwaschpulver ist.

13. Verwendung einer Reinigungsmittelzusammensetzung gemäß einem der Ansprüche 1 bis 11, die eine Textilwaschflüssigkeit ist.

## Revendications

1. Utilisation d'un produit détergent déodorant pour laver des étoffes afin de conférer auxdites étoffes la capacité de faire disparaître les odeurs corporelles désagréables quand on porte l'étoffe sous forme d'un vêtement, le produit détergent comprenant :
(i) de 5 à 95% en poids si le produit est solide, de 5 à 80% en poids si le produit est un liquide, d'un composé détergent actif non savonneux choisi parmi les composés détergents actifs anioniques, non ioniques, cationiques, amphotères et zwitterioniques ;
(ii) de 0,05 à 3% en poids d'une composition déodorante comprenant une composition de parfums qui abaissent la tension partielle de vapeur de la morpholine d'au moins 10% de plus que la valeur requise par la Loi de Raoult, la composition déodorante ayant un Indice Déodorant (mesuré par le test de Whitehouse et Carter modifié par l'emploi d'une échelle de 0 à 5 et la détermination après 5 heures au lieu de 24 heures), quand on l'incorpore dans un pain de toilette en une concentration de 1,5 en poids, de 0,50 à 3,5 ; et
(iii) d'autres additifs aux détergents qui comportent un adjuvant pour former le complément du produit ;
le produit détergent ayant un Indice de Réduction d'Odeur d'au moins 0,50, l'Indice de Réduction d' Odeur étant défini par le Test d'Indice de Réduction d' Odeur qui est lui-même à base du Test de Whitehouse et Carter avec les modifications suivantes, à savoir qu'on détermine les mauvaises odeurs corporelles 5 heures après le traitement au lieu de 24 heures, en utilisant une échelle d'odeurs de 0 à 5 au lieu de 0 à 10, le Test étant effectué après application à une étoffe pour chemise au cours du cycle de lavage d'un procédé de blanchisserie, les mauvaises odeurs corporelles qui demeurent sur l'étoffe de la chemise étant déterminées plutôt que les mauvaises odeurs des aisselles elles-mêmes, le lavage des étoffes et la détermination d'odeur se faisant comme suit:
(a) on soumet à un prélavage des chemises à boutonnage intégral du type polyester dans une machine à laver automatique en utilisant une poudre détergente non ionique de lavage d'étoffe pour assurer que les chemises à utiliser dans le Test d'indice de Réduction d'Odeur sont toutes pareillement propres et exemptes d'apprêt avant le lavage dans le produit détergent ;
(b) on sèche les chemises prélavées et on les lave de nouveau dans une machine à laver automatique dans laquelle la liqueur de lavage contient 0,4% en poids de la poudre de lavage d'étoffe ou du produit liquide testé contenant 0,2% en poids de composition déodorante, le rapport de l'étoffe à chemise (en poids sec) à la liqueur de lavage étant de 40 g d'étoffe par litre de liqueur de lavage ;
(c) on agite les chemises dans la liqueur de lavage pendant 10 minutes à 50°C , puis on rince et on essore jusqu'à une teneur en humidité d'environ 50% en poids d'eau, et finalement on sèche jusqu'à une teneur d'humidité ne dépassant pas 10% pour obtenir des chemises de test ;
(d) on lave de nouveau un autre lot de chemises prélavées pour servir de chemises-témoins et on sèche dans les mêmes conditions sauf qu'on n'admet pas de composition déodorante dans la poudre de lavage ou le produit liquide qu'on ajoute à la liqueur de lavage ;
(e) on plie les chemises et on les range pendant une nuit dans des sacs de polyéthylène jusqu'au moment de les faire tester par un groupe d'hommes, la moitié de ces hommes portant des chemises testées qui ont été traitées par la composition déodorante dans la poudre de lavage ou dans le produit liquide et l'autre moitié portant des chemises-témoins n'ayant pas subi de traitement par la composition déodorante ; et
(f) après 5 heures, on enregistre l'odeur de l'étoffe dans la zone des aisselles, cette opération étant faite dans chaque cas par des femmes, l'Indice de Réduction d'Odeur de la poudre de lavage ou du produit liquide étant finalement calculé comme la différence entre la note moyenne pour les chemises lavées avec le produit du test et la note moyenne pour les chemises lavées par le produit-témoin.

2. Utilisation d'un produit détergent selon la revendication 1, dans laquelle le composé détergent actif non savonneux comprend un composé détergent anionique choisi parmi :
(a) les alkylsulfates de sodium et de potassium qu'on obtient en sulfatant les alcools en C₈₋₁₈ provenant de la réduction des glycérides de suif ou d'huile de coprah ;
(b) les alkylbenzènesulfonates de sodium et de potassium dont le radical alkyle contient de 9 à 15 atomes de carbone sous forme d'une chaîne droite ou ramifiée ;
(c) les alkyl-glycérol-éther-sulfonates de sodium ;
(d) les sulfonates et sulfates de sodium des monoglycérides d'acides gras d'huile de coprah ;
(e) les alkylphénoléthylène-oxyde-éthersulfates de sodium ou de potassium contenant de 1 à 10 motifs d' oxyde d'éthylène par molécule et dont les radicaux alkyle contiennent de 8 à 12 atomes de carbone ;
(f) les sels hydrosolubles d'esters d'acides gras alpha-sulfonés contenant de 6 à 20 atomes de carbone dans le groupe ester ;
(g) les sels hydrosolubles d'acides 2-acyloxy-alcane-1-sulfoniques contenant de 2 à 9 atomes de carbone dans le radical acyle et de 9 à 23 atomes de carbone dans le fragment alcane ;
(h) les alkyléthersulfates contenant de 10 à 20 atomes de carbone dans le radical alkyle et de 1 à 30 moles d'oxyde d'éthylène ;
(i) les sels hydrosolubles d'oléfinosulfonates contenant de 12 à 24 atomes de carbone ; et
(j) les 6-alkyloxyalcanesulfonates contenant de 1 à 3 atomes de carbone dans le radical alkyle et de 8 à 20 atomes de carbone dans le fragment alcane.

3. Utilisation d'un produit détergent selon la revendication 1, dans laquelle le composé détergent actif non savonneux comprend un composé détergent actif anionique choisi parmi :
(a) les alkylbenzènesulfonates linéaires à chaîne droite contenant de 11 à 14 atomes de carbone dans le radical alkyle ;
(b) les suif-alkylsulfates en C₁₂₋₂₀ ;
(c) les coprah-alkylglycérylsulfonates ; et
(d) les alkyléthersulfates dont le fragment alkyle contient de 14 à 18 atomes de carbone et le degré moyen d'éthoxylation est de 1 à 6.

4. Utilisation d'un produit détergent selon la revendication 1, dans laquelle le composé détergent actif non savonneux comprend un composé détergent actif. choisi parmi :
(a) un alkylbenzènesulfonate linéaire de sodium en C₁₀₋₁₂ ;
(b) un alkylbenzènesulfonate de triéthanolamine en C₁₀₋₁₂ ;
(c) un suif-alkylsulfate de sodium ;
(d) un coprah-alkylglycéryléthersulfonate de sodium ; et
(e) un sel sodique d'un produit sulfoné de condensation de suif-alcool avec 3 à 10 moles d'oxyde d' éthylène.

5. Utilisation d'un produit détergent selon l'une quelconque des revendications précédentes, dans laquelle le composé détergent actif non savonneux comprend un détergent actif non ionique choisi parmi :
(a) des produits de condensation d'alcools aliphatiques à chaîne droite ou ramifiée ou d'acides carboxyliques de 8 à 18 atomes de carbone avec l'oxyde d' éthylène ;
(b) les produits de condensation d'alkylphénols dont le radical alkyle contient de 6 à 12 atomes de carbone avec 5 à 25 moles d'oxyde d'éthylène par mole d'alkylphénol ;
(c) les produits de condensation du produit de réaction d'éthylène-diamine et d'oxyde de propylène avec l'oxyde d'éthylène, les produits de condensation contenant de 40 à 80% de radicaux polyoxyéthylène en poids et ayant une masse moléculaire de 5000 à 11.000 ;
(d) les oxydes d'amines tertiaires de structure R₃NO dans laquelle un radical R est un radical alkyle de 8 à 18 atomes de carbone et les autres sont chacun un radical méthyle, éthyle ou hydroxyéthyle ;
(e) les oxydes de phosphines tertiaires de structure R₃PO dans laquelle un radical R est un radical alkyle de 10 à 18 atomes de carbone et les autres sont chacun un radical alkyle ou hydroxyalkyle de 1 à 3 atomes de carbone ; et
(f) les dialkylsulfoxydes de structure R₂SO, dans laquelle un radical R est un radical alkyle de 10 à 18 atomes de carbone et l'autre est le radical méthyle ou éthyle.

6. Utilisation d'un produit détergent selon l'une quelconque des revendications précédentes, dans laquelle la composition déodorante forme de 0,1 à 1% du poids du produit.

7. Utilisation d'un produit détergent selon l'une quelconque des revendications précédentes dans laquelle l'adjuvant est choisi parmi le tripolyphosphate de sodium, le pyrophosphate de sodium et le pyrophosphate de potassium.

8. Utilisation d'un produit détergent selon l'une quelconque des revendications précédentes, dans laquelle les autres additifs aux détergents comprennent un agent de blanchiment.

9. utilisation d'un produit détergent selon la revendication 8, dans laquelle l'agent de blanchiment comprend un perborate.

10. Utilisation d'un produit détergent selon l'une quelconque des revendications précédentes, dans laquelle les autres additifs aux détergents comprennent une enzyme.

11. Utilisation d'un produit détergent selon l'une quelconque des revendications précédentes, dans laquelle les autres additifs aux détergents comprennent un agent de fluorescence.

12. Utilisation d'un produit détergent selon l'une quelconque des revendications précédentes, qui est une poudre de lavage d'étoffe.

13. Utilisation d'un produit détergent selon l'une quelconque des revendications 1 à 11, qui est un liquide de lavage d'étoffe.
